# EUROPEAN PATENT APPLICATION

(11) **EP 2 258 864 A1**
(43) Date of publication of application: **08.12.2010**
(21) Application number: 09161881.9
(22) Date of filing: 03.06.2009
(51) Int. Cl.: C12Q 1/68

(54) **Genetic marker for use in the treatment of leukemia**

(71) Applicant: Medizinische Hochschule Hannover, 30625 Hannover (DE)
(72) Inventor: Damm, Frederik Martin Georges, 30451 Hannover (DE); Heuser, Michael, 30655 Hannover (DE); Ganser, Arnold, 30559 Hannover (DE)
(74) Representative: Taruttis, Stefan Georg

(57) **Abstract**

The invention relates to the use of a genetic marker in a process for analysis of leukemia patients, especially for use in the prediction of the suitability of chemotherapy in a group of leukemia patients. Further, the invention relates to chemotherapeutical agents for use in the therapy of leukemia patients that have been diagnosed to be at least heterozygous for the relevant allele of the genetic marker.

SEQUENCE LISTING

<110>  Medizinische Hochschule Hannover

<120>  Genetic marker for use in the treatment of leukemia

<130>  M1018EP

<160>  3

<170>  PatentIn version 3.5

<210>  1
<211>  3029
<212>  DNA
<213>  Homo sapiens

<220>
<221>  exon
<222>  (197)..(1750)
<223>  exon 7

<220>
<221>  misc_feature
<222>  (1303)..(1303)
<223>  SNP ID rs16754

<400>  1
ccaggcagct ggggtaagga gttcaaggca gcgcccacac ccgggggctc tccgcaaccc      60

gaccgcctgt ccgctccccc acttcccgcc ctccctccca cctactcatt cacccaccca     120

cccacccaga gccgggacgg cagcccaggc gcccgggccc cgccgtctcc tcgccgcgat     180

cctggacttc ctcttg ctg cag gac ccg gct tcc acg tgt gtc ccg gag ccg     232
                  Leu Gln Asp Pro Ala Ser Thr Cys Val Pro Glu Pro
                  1               5                   10

gcg tct cag cac acg ctc cgc tcc ggg cct ggg tgc cta cag cag cca     280
Ala Ser Gln His Thr Leu Arg Ser Gly Pro Gly Cys Leu Gln Gln Pro
        15                  20                  25

gag cag cag gga gtc cgg gac ccg ggc ggc atc tgg gcc aag tta ggc     328
Glu Gln Gln Gly Val Arg Asp Pro Gly Gly Ile Trp Ala Lys Leu Gly
    30                  35                  40

gcc gcc gag gcc agc gct gaa cgt ctc cag ggc cgg agg agc cgc ggg     376
Ala Ala Glu Ala Ser Ala Glu Arg Leu Gln Gly Arg Arg Ser Arg Gly
45                  50                  55                  60

gcg tcc ggg tct gag ccg cag caa atg ggc tcc gac gtg cgg gac ctg     424
Ala Ser Gly Ser Glu Pro Gln Gln Met Gly Ser Asp Val Arg Asp Leu
                65                  70                  75

aac gcg ctg ctg ccc gcc gtc ccc tcc ctg ggt ggc ggc ggc ggc tgt     472
Asn Ala Leu Leu Pro Ala Val Pro Ser Leu Gly Gly Gly Gly Gly Cys
            80                  85                  90

gcc ctg cct gtg agc ggc gcg gcg cag tgg gcg ccg gtg ctg gac ttt     520
Ala Leu Pro Val Ser Gly Ala Ala Gln Trp Ala Pro Val Leu Asp Phe
        95                  100                 105

gcg ccc ccg ggc gct tcg gct tac ggg tcg ttg ggc ggc ccc gcg ccg     568
Ala Pro Pro Gly Ala Ser Ala Tyr Gly Ser Leu Gly Gly Pro Ala Pro
    110                 115                 120

cca ccg gct ccg ccg cca ccc ccg ccg ccg ccg cct cac tcc ttc atc     616
Pro Pro Ala Pro Pro Pro Pro Pro Pro Pro Pro Pro His Ser Phe Ile

125                     130                     135                     140

aaa cag gag ccg agc tgg ggc ggc gcg gag ccg cac gag gag cag tgc          664
Lys Gln Glu Pro Ser Trp Gly Gly Ala Glu Pro His Glu Glu Gln Cys
                145                     150                     155

ctg agc gcc ttc act gtc cac ttt tcc ggc cag ttc act ggc aca gcc          712
Leu Ser Ala Phe Thr Val His Phe Ser Gly Gln Phe Thr Gly Thr Ala
            160                     165                     170

gga gcc tgt cgc tac ggg ccc ttc ggt cct cct ccg ccc agc cag gcg          760
Gly Ala Cys Arg Tyr Gly Pro Phe Gly Pro Pro Pro Pro Ser Gln Ala
        175                     180                     185

tca tcc ggc cag gcc agg atg ttt cct aac gcg ccc tac ctg ccc agc          808
Ser Ser Gly Gln Ala Arg Met Phe Pro Asn Ala Pro Tyr Leu Pro Ser
    190                     195                     200

tgc ctc gag agc cag ccc gct att cgc aat cag ggt tac agc acg gtc          856
Cys Leu Glu Ser Gln Pro Ala Ile Arg Asn Gln Gly Tyr Ser Thr Val
205                     210                     215                     220

acc ttc gac ggg acg ccc agc tac ggt cac acg ccc tcg cac cat gcg          904
Thr Phe Asp Gly Thr Pro Ser Tyr Gly His Thr Pro Ser His His Ala
                225                     230                     235

gcg cag ttc ccc aac cac tca ttc aag cat gag gat ccc atg ggc cag          952
Ala Gln Phe Pro Asn His Ser Phe Lys His Glu Asp Pro Met Gly Gln
            240                     245                     250

cag ggc tcg ctg ggt gag cag cag tac tcg gtg ccg ccc ccg gtc tat          1000
Gln Gly Ser Leu Gly Glu Gln Gln Tyr Ser Val Pro Pro Pro Val Tyr
        255                     260                     265

ggc tgc cac acc ccc acc gac agc tgc acc ggc agc cag gct ttg ctg          1048
Gly Cys His Thr Pro Thr Asp Ser Cys Thr Gly Ser Gln Ala Leu Leu
    270                     275                     280

ctg agg acg ccc tac agc agt gac aat tta tac caa atg aca tcc cag          1096
Leu Arg Thr Pro Tyr Ser Ser Asp Asn Leu Tyr Gln Met Thr Ser Gln
285                     290                     295                     300

ctt gaa tgc atg acc tgg aat cag atg aac tta gga gcc acc tta aag          1144
Leu Glu Cys Met Thr Trp Asn Gln Met Asn Leu Gly Ala Thr Leu Lys
                305                     310                     315

gga gtt gct gct ggg agc tcc agc tca gtg aaa tgg aca gaa ggg cag          1192
Gly Val Ala Ala Gly Ser Ser Ser Ser Val Lys Trp Thr Glu Gly Gln
            320                     325                     330

agc aac cac agc aca ggg tac gag agc gat aac cac aca acg ccc atc          1240
Ser Asn His Ser Thr Gly Tyr Glu Ser Asp Asn His Thr Thr Pro Ile
        335                     340                     345

ctc tgc gga gcc caa tac aga ata cac acg cac ggt gtc ttc aga ggc          1288
Leu Cys Gly Ala Gln Tyr Arg Ile His Thr His Gly Val Phe Arg Gly
    350                     355                     360

att cag gat gtg cga cgt gtg cct gga gta gcc ccg act ctt gta cgg          1336
Ile Gln Asp Val Arg Arg Val Pro Gly Val Ala Pro Thr Leu Val Arg
365                     370                     375                     380

tcg gca tct gag acc agt gag aaa cgc ccc ttc atg tgt gct tac cca          1384
Ser Ala Ser Glu Thr Ser Glu Lys Arg Pro Phe Met Cys Ala Tyr Pro
                385                     390                     395

ggc tgc aat aag aga tat ttt aag ctg tcc cac tta cag atg cac agc          1432
Gly Cys Asn Lys Arg Tyr Phe Lys Leu Ser His Leu Gln Met His Ser
            400                     405                     410

agg aag cac act ggt gag aaa cca tac cag tgt gac ttc aag gac tgt     1480
Arg Lys His Thr Gly Glu Lys Pro Tyr Gln Cys Asp Phe Lys Asp Cys
        415                 420                 425

gaa cga agg ttt tct cgt tca gac cag ctc aaa aga cac caa agg aga     1528
Glu Arg Arg Phe Ser Arg Ser Asp Gln Leu Lys Arg His Gln Arg Arg
    430                 435                 440

cat aca ggt gtg aaa cca ttc cag tgt aaa act tgt cag cga aag ttc     1576
His Thr Gly Val Lys Pro Phe Gln Cys Lys Thr Cys Gln Arg Lys Phe
445                 450                 455                 460

tcc cgg tcc gac cac ctg aag acc cac acc agg act cat aca ggt aaa     1624
Ser Arg Ser Asp His Leu Lys Thr His Thr Arg Thr His Thr Gly Lys
                465                 470                 475

aca agt gaa aag ccc ttc agc tgt cgg tgg cca agt tgt cag aaa aag     1672
Thr Ser Glu Lys Pro Phe Ser Cys Arg Trp Pro Ser Cys Gln Lys Lys
            480                 485                 490

ttt gcc cgg tca gat gaa tta gtc cgc cat cac aac atg cat cag aga     1720
Phe Ala Arg Ser Asp Glu Leu Val Arg His His Asn Met His Gln Arg
        495                 500                 505

aac atg acc aaa ctc cag ctg gcg ctt tga ggggtctccc tcggggaccg     1770
Asn Met Thr Lys Leu Gln Leu Ala Leu
    510                 515

ttcagtgtcc caggcagcac agtgtgtgaa ctgctttcaa gtctgactct ccactcctcc     1830

tcactaaaaa ggaaacttca gttgatcttc ttcatccaac ttccaagaca agataccggt     1890

gcttctggaa actaccaggt gtgcctggaa gagttggtct ctgccctgcc tacttttagt     1950

tgactcacag gccctggaga agcagctaac aatgtctggt tagttaaaag cccattgcca     2010

tttggtgtgg attttctact gtaagaagag ccatagctga tcatgtcccc ctgacccttc     2070

ccttcttttt ttatgctcgt tttcgctggg gatggaatta ttgtaccatt ttctatcatg     2130

gaatatttat aggccagggc atgtgtatgt gtctgctaat gtaaactttg tcatggtttc     2190

catttactaa cagcaacagc aagaaataaa tcagagagca aggcatcggg ggtgaatctt     2250

gtctaacatt cccgaggtca gccaggctgc taacctggaa agcaggatgt agttctgcca     2310

ggcaactttt aaagctcatg catttcaagc agctgaagaa aaaatcagaa ctaaccagta     2370

cctctgtata gaaatctaaa agaattttac cattcagtta attcaatgtg aacactggca     2430

cactgctctt aagaaactat gaagatctga gatttttttg tgtatgtttt tgactctttt     2490

gagtggtaat catatgtgtc tttatagatg tacatacctc cttgcacaaa tggaggggaa     2550

ttcattttca tcactgggag tgtccttagt gtataaaaac catgctggta tatggcttca     2610

agttgtaaaa atgaaagtga ctttaaaaga aaatagggga tggtccagga tctccactga     2670

taagactgtt tttaagtaac ttaaggacct ttgggtctac aagtatatgt gaaaaaaatg     2730

agacttactg ggtgaggaaa tccattgttt aaagatggtc gtgtgtgtgt gtgtgtgtgt     2790

gtgtgtgtgt gtgttgtgtt gtgttttgtt ttttaaggga gggaatttat tatttaccgt     2850

tgcttgaaat tactgtgtaa atatatgtct gataatgatt tgctctttga caactaaaat     2910

taggactgta taagtactag atgcatcact gggtgttgat cttacaagat attgatgata     2970

acacttaaaa ttgtaacctg catttttcac tttgctctca attaaagtct attcaaaag      3029

<210>  2
<211>  21
<212>  DNA
<213>  Artificial Sequence

<220>
<223>  SEQ ID No. 2, primer

<400>  2
ctccagtgct cactctccct c                                                21

<210>  3
<211>  21
<212>  DNA
<213>  Artificial Sequence

<220>
<223>  SEQ ID No. 3, primer

<400>  3
ccttagcagt gtgagagcct g                                                21

## Description

The invention relates to the use of a genetic marker in a process for analysis of leukemia patients for use in therapy, especially for use in the prediction of the suitability of chemotherapy in a group of leukemia patients on the basis of their alleles of the marker. Further, the invention relates to chemotherapeutical agents for use in the therapy of leukemia patients that have been diagnosed to be at least heterozygous for the relevant allele of the genetic marker. Further, the invention relates to a pharmaceutical composition containing at least one known chemotherapeutical agent specified for use in the treatment of leukemia, especially for the treatment of leukemia patients being diagnosed to be homozygous for the relevant allele of the marker, especially for leukemia patients who have been identified to have a genotype that is considered a high-risk leukemia genotype, including, but not restricted to CN-AML.

Hollink et al. in Blood, 2008 - 09-177949 describe that 24% of diagnostic samples obtained from children suffering from AML were analysed to have an A to G mutation in a mutational hotspot of exon 7 of the Wilms' tumour (WT1) gene.

Milani et al. in Nuc. Acids Res. 35(5) (2007) investigated the gene expression of marker allele expression in cultivated cell lines.

In leukemia patients, especially in patients diagnosed with acute myeloid leukemia (AML), or diagnosed with acute lymphoblastic leukemia (ALL), especially for these leukemia patients having a normal cytogenetic characterisation (CN-AML), to-date a classification into risk classes is made on the basis of the presence or absence of molecular markers. The classification of leukemia patients into the high risk group can e.g. be made on the basis of the presence of the FLT3-ITD marker, and/or on the presence of wild-type NPM1 in combination with wild-type FLT3, presence of wild-type NPM1 in combination with FLT3-ITD mutation, or presence of mutated NPM1 in combination with (/) FLT3-ITD mutation, including patients having a caryotype without aberrations, i.e. a normal caryotype (cytogenetically normal acute myeloid leukemia (CN-AML)). The high risk leukemia patients are currently treated by intensive care therapy, including stem cell transplantation. Due to the high toxicity of intensive care therapy, a mortality of up to 30% as caused by intensive therapy side effects can be observed.

In addition to Hollink et al, Paschka et al. (J. Clin. Oncol. 26, 4595-4602 (2008)) and Virappane et al. (J. Clin. Oncol. 26, 5429-5435 (2008)) describe that acquired mutations of the WT1 gene are associated with poor prognosis in adult and pediatric CN - AML patients. These findings were contradicted by a large study of 617 patients (17) by the finding that mutations in WT1 were not related to prognosis in CN - AML patients. In addition to the contradictory findings of the relevance of mutations in the WT1 gene, previous reports are contradictory in respect of the prognostic value of WT1 mRNA expression on the prognosis of AML - treatment. For example, Bergmann et al. (Blood, 90: 1217-1225 (1997)), Weisser et al. (Leukemia, 19: 1416-23 (1005)), Lipillonne (J. Clin. Oncol. 24: 1507-15 (2006)), and Inoue (Blood, 84: 3071-3079 (1994)) observed expression of WT1 in 75 - 100% of AML patients, with high levels of WT1 mRNA being associated with a favourable prognosis of AML treatment. In contrast, Gaiger et al. (Leukemia, 12: 1886-94 (1998)), Schmid et al. (Leukemia 11: 639-43 (1997)), and Rodrigues et al. (Prediatr. Blood Cancer 49: 133-8 (2007)) did not find any correlation of WT1 expression levels to the outcome of AML treatment, and hence no prognostic value of WT1 expression levels.

### Objects of the invention

It is an object of the present invention to provide an analytical method which is useful for the identification of patients, especially patients classified as high risk leukemia patients, who can be treated successfully by standard chemotherapy, especially by chemotherapy only, e.g. without stem cell transplantation, instead of intensive treatment generally used for high risk leukemia patients, which intensive treatment can involve stem cell therapy. Classification into the high-risk class can e.g. be done as described above.

As a further object, the present invention seeks to provide a pharmaceutical composition for use in the treatment of leukemia, especially for leukemia in patients who are classified as high risk patients, the composition comprising chemotherapeutical agents, as well as the use of chemotherapeutical agents for the production of pharmaceutical compositions for use in treatment of leukemia in high risk leukemia patients.

### General description of the invention

The invention attains the above-mentioned objects by providing a sub-group of leukemia patients, especially of high risk leukemia patients, especially high risk leukemia patients diagnosed for AML or ALL, preferably CN-AML, and by providing an analytical method for use in the identification of a sub-group of high risk leukemia patients, which sub-group of high risk leukemia patients can be treated successfully by chemotherapy, especially using chemotherapeutical agents which are today used for the treatment of low risk or intermediate risk leukemia patients. Further, the invention provides for a process for analysis of the sub-group of patients, and for the use of chemotherapeutical agents for the production of pharmaceutical compositions for the treatment of leukemia, wherein the patients are identified, e.g. by the analytical method of the invention, as the sub-group of high-risk leukemia patients who at least heterozygously carry the minor allele of the marker.

It has been found that in high risk leukemia patients, who are defined in accordance with the classification as used to-date, especially leukemia patients who have been diagnosed positive for wild-type NPM1 in combination with wild-type FLT3 (FLT3-ITD^{negative}), or wild-type NPM1 in combination with the FLT3-ITD mutation, or mutated NPM1 in combination with the FLT3-ITD mutation, or MLL partial tandem duplication (PTD) mutation, or mutations of NPM1 and FLT3-ITD as well as CEBPA, including in each case cytogenetically normal AML (CN-AML), one allele of the marker SNP ID: rs16754 is a suitable prognostic indicator of the outcome of a chemotherapy in the treatment of this sub-group of patients, i.e. without stem cell transplantation therapy of this sub-group of high-risk leukemia patients.

It has been found that the allele containing a G in marker SNP ID: rs16754, also termed minor allele for the purposes of the present invention, is coupled to a positive prognosis of treatment by chemotherapy also for high risk leukemia patients. Accordingly, high risk leukemia patients as defined above, including CN-AML patients, who e.g. carry a FLT3- ITD mutation, with or without an NPM1 mutation, or who have wild-type FLT3 / wild-type NPM1 (no FLT3-ITD mutation), if analysed to carry the minor (G) allele of the marker SNP ID: rs16754 heterozygously or homozygously, can be identified by the analytical process according to the invention, and can be treated with a positive prognosis by chemotherapy as e.g. used currently for low risk and intermediate risk leukemia patients, preferably excluding stem cell transplantation. Therefore, the present invention provides for a selection process of a sub-group of high-risk leukemia patients, which sub-group can be treated with a positive prognosis by chemotherapy, while avoiding the severe side effects of the treatment currently applied to high risk patients, which sub-group is characterized by the minor allele of the marker SNP ID: rs16754. The marker including the reference number of a single nucleotide polymorphism (Ref SNP ID) rs16754 is e.g. available at NCBI data bank (build 129), and is also termed C1107 A>G at GenBank entry NM/024426.3 (NM-024426.3: c.1107A>G). The enclosed nucleotide sequence SEQ ID No. 1 is the WT1 gene according to Refseq. NM024426, containing at nucleotides 197 to 1750 the coding sequence, exon 7 at nucleotides 1295 to 1445, and marker SNP ID: rs16754 at position 1303, which corresponds to nucleotide 1107 of the coding sequence section (nt 197 to 1750).

Consequently, the use of the analytical method of the present invention, by identifying from the group of high risk leukemia patients a sub-group on the basis of the minor allele of the marker allows to use chemotherapeutical treatment to this sub-group of high risk leukemia patients with a positive prognosis, instead of subjecting the sub-group of patients to high risk intensive treatments generally used for the treatment of the group of high risk patients. Accordingly, the invention allows to reduce the overall mortality of high risk leukemia patients which is caused by side-effects of intensive treatment, in combination with a better prospect of healing for this sub-group of high risk leukemia patients by selecting the patients which are at least heterozygous for the minor allele of marker SNP: ID rs16754. In accordance with the positive prognosis of chemotherapy for this sub-group of patients, the invention also provides for the use of chemotherapeutical agents for the production of pharmaceutical compositions for use in the treatment of leukemia, especially in high risk patients, wherein the patients are characterized in having the minor allele of the marker SNP ID: rs16754 at least heterozygously.

Accordingly, the invention provides a pharmaceutical composition comprising at least one chemotherapeutical agent suitable for treatment of leukemia, which pharmaceutical composition is accompanied by instructions for use indicating that the composition is suitable for leukemia patients of the sub-group, i.e. suitable for use in the treatment of leukemia patients, especially high-risk leukemia patients, preferably CN-AML, which patients are diagnosed to have the minor allele of the marker SNP ID: rs16754, i.e. heterozygously (A/G) or homozygously (G/G).

Further, the invention provides a process for the treatment of leukemia in human patients by a pharmaceutical composition comprising at least one chemotherapeutical agent, which process is for patients who at least heterozygously carry the allele G of the marker SNP ID: rs16754. In accordance with the selection of the sub-group of high-risk leukemia patients for a treatment consisting of chemotherapy, i.e. excluding stem cell therapy, the invention also provides for a process for the treatment of leukemia patients, comprising the steps of analysing the alleles of the marker SNP ID: rs16754, and applying a treatment consisting of chemotherapy to patients who are diagnosed to carry the minor allele of the marker SNP ID: rs16754 at least heterozygously. Alternatively or additionally, the process comprises the step of applying an intensive chemotherapeutical treatment comprising stem cell therapy to patients who are diagnosed to carry the major allele of marker SNP ID: rs16754 homozygously.

The preferred pharmaceutical agent for use in the present invention for the treatment of high risk leukemia patients having the minor allele of the marker at least in a heterozygous state comprises idarubicin, etoposide , cytarabine, fludarabine, G -CSF, daunorubicin, e.g. in chemotherapeutical regimens comprising FLAG-Ida (chemotherapy with fludarabine, cytarabine, G-CSF, and idarubicin), and high-dose cytarabine (HD-AraC).

### Detailed description of the invention

The invention is now described in greater detail with reference to the figures, wherein
- Figure 1 shows A) the relapse-free survival of high risk patients in relation to presence of the minor and major allele of the marker, respectively, B) the overall survival of high risk patients having the minor and major allele of the marker, respectively, C) the relapse-free survival of low risk patients having the minor and major allele of the marker, respectively, and D) the overall survival of low risk patients with the minor and major allele of the marker, respectively,
- Figure 2 shows A) the relapse-free survival rate and B) the overall survival rate for patients having the major marker allele for wild-type or mutated WT1, and
- Figure 3 shows the dependency of A) the relapse-free survival and B) overall survival on WT1 mutation for CN-AML patients having the major allele of the marker homozygously.

In the following analysis, cytogenetically normal acute myeloid (CN-AML) leukemia patients were investigated by analysing mutations in the Wilm's tumor 1 (WT1) gene, and analysing WT1 gene expression. It has been found that the marker SNP ID: rs16754 (databank SNP build 129) of WT1 can be used as a prognostic marker for the outcome of chemotherapy in CN - AML patients, namely that patients carrying the minor allele at least heterozygously, including high risk leukemia patients, have a positive prognosis for chemotherapy. This positive prognosis also of high-risk leukemia patients for chemotherapy, preferably for chemotherapy without stem cell therapy, on the basis of the allele of SNP ID:
rs16754 is unknown in the field, and allows a more specific treatment of leukemia patients.

Accordingly, patients having the major allele of the marker SNP ID: rs16754 homozygously cannot be treated by chemotherapy alone with a positive prognosis, and it is therefore preferred to assign these patients to high intensity treatment including stem cell therapy.

In the study providing the basis for the present invention, diagnostic bone marrow or peripheral blood samples obtained from 249 adult patients with *de novo* or secondary AML (classified as M0 - M2, M4 - M7 in the French - American - British classification) at normal cytogenetics were analysed. All patients received intensive, response-adapted double induction and consolidation chemotherapy after written informed consent prior to therapy, with approval by the institutional review board of the Medizinische Hochschule Hannover.

Generally, mononuclear cells from samples were enriched by Ficoll density gradient centrifugation. Preferably, samples were analysed by G- and R-banding analysis and by fluorescence *in situ* hybridisation (FISH) using a comprehensive DNA probe set, as e.g. described in Metzke-Heidemann et al. (Genes Chromosomes Cancer 31: 10-14 (2001)).

Genomic DNA was extracted for analysis using ion exchange purification using the AllPrep DNA/RNA kit (Qiagen, Germany) according to the manufacturer's instructions. For amplification of sections of the WT1 gene, the polymerase chain reaction (PCR) was used, followed by purification of amplification products and their direct sequencing using dideoxy nucleotide termination cycle sequencing and analysis on an automated detection device, a 3130 GeneticAnalyser (AppliedBiosystems, Germany) with automatic fragment detection. For confirmation of point mutations, including insertions, deletions or duplications, fragments were confirmed by sub-cloning in vector pGEM-T (obtainable from Promega, Germany). For RNA analysis, cDNA was generated by M-MLV reverse transcriptase (obtainable from Invitrogen) with random hexamer priming, coupled with PCR, using the WT1 ProfileQuant kit (Ipsogen, France) according to the manufacturer's instructions. The analysis of mutation FLT3-ITD was according to Frohling et al. (Blood 100: 4372-4380 (2002)), of MLL-PTD according to Whitman et al. (Blood 106, 345-52 (2005)) or Caligiuri et al. (Cancer Res. 58:55-9 (1998)), of NPM1 according to Doehner et al. (Blood 106: 3740-3746 (2005)), and of CEBPA according to Frohling et al. (J. Clin. Oncol. 22: 624-633 (2004)).

For PCR analysis, a fragment of exon 7 containing the marker SNP ID: rs16754 was amplified from genomic patient DNA using forward primer CTCCAGTGCTCACTCTCCCTC (Seq ID No. 2), and reverse primer CCTTAGCAGTGTGAGAGCCTG (Seq ID No. 3). The amplification product was purified and sequenced, e.g. using a 3130 Genetic Analyzer (ABI).

Statistical analysis was in accordance with Heuser et al. (Blood 108: 3898-3905 (2006), or Blood 110: 1639-47 (2007)), defining complete remission (CR), relapse-free survival (RFS), and overall survival (OS) in accordance with recommended criteria (Cheson et al., J. Clin. Oncol. 21: 4642-4649 (2003)), using the statistical programme SPSS16.0 (obtained from SPSS Science, Chicago, USA).

For WT1 expression analysis, samples were classified into 2 groups with respect to the median value of WT1 expression (median normalized copy number, transcript number of WT1 per ABL-transcript: 1.171, 0.0011 - 44.84).

For pairwise comparison, the Kolmogorov-Smirnov test for continuous variables and the Chi-squared test for categorical variables was made. Estimation of distribution of RFS and OS was made by the Kaplan-Meier method, and comparison of differences of survival curves by the log-rank test. For multivariate analysis, a Cox proportional hazards model was constructed for RFS and OS, adjusting for potential confounding covariates using the forward-selection method of Cox, J. R. Stat. Soc. B 34: 187-202 (1972). As variables for model inclusion were considered age, hemoglobin, platelet count, blast percentage in peripheral blood or bone marrow (above or below median), sex, ECOG performance status (0, 1, or 2, respectively), presence of a mutation in NPM1 and wild-type FLT3 as one group, presence of mutations in CEBPA, MLL-PTD, and in WT1, high or low WT1 expression, and presence of mutation in SNP ID: rs16754 (homozygous for major allele vs. heterozygous or homozygous for minor allele). Variables with P of 0.1 or less in univariate analysis for RFS or OS were included in the statistical model. The two-sided level of significance was set at P below 0.05.

It has been found that patients having the minor allele of SNP ID: rs16754 at least heterozygously in comparison to patients who are homozygous for the major allele of SNP ID: rs16754 show no significant difference in baseline characteristics, treatment regimens, or in the frequencies of mutations in FLT3-ITD (P= 0.4, 28% vs. 34%), NPM1 (P=0.96, 59% vs. 58%), CEBPA (P=0.36, 19% vs. 14%), MLL-PTD (P=0.35, 2% vs. 4%), WT1 (P=0.25, 16% vs. 10%), or the low risk mutation status of mutation in NPM1 in combination with no FLT3-ITD (P=0.87, 36% vs. 35%). Comparison of patients having a mutation in WT1 to patients with wild-type WT1 also showed no significant difference in baseline characteristics (except for a lower hemoglobin level in patients with a WT1 mutation), treatment modalities, and frequencies of further mutations. The expression levels of WT1 were similar in patients with minor allele of SNP ID: rs16754 and patients homozygous for the major allele of SNP ID: rs16754, as well as for patients with or without WT1 mutations (e.g. mutations in the WT1 gene except for marker SNP ID: rs16754, e.g. further acquired mutations).

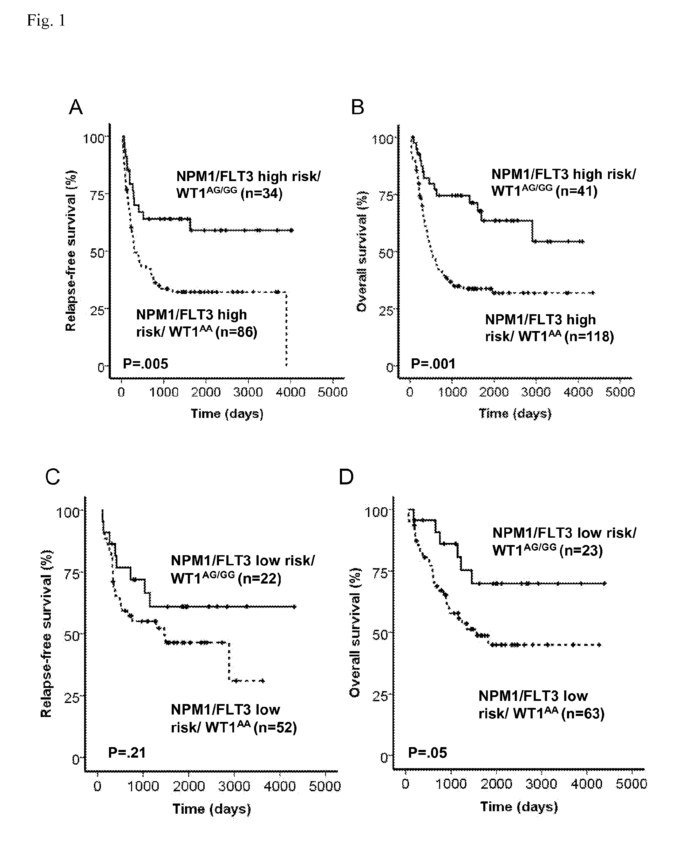

The significance of the allele of the marker SNP ID: rs16754 is shown in Figure 1, wherein the comparison of A) the relapse-free (RFS) and B) the overall survival (OS) for patients who are classified in accordance with wild-type NPM1 and FLT3-ITD as high risk, is shown for patients who are diagnosed as heterozygous (A/G) and homozygous (G/G) for the minor allele of marker SNP ID: rs16754 and for patients diagnosed as homozygous (AA) for the major allele of marker SNP ID: rs16754. It is evident that the at least heterozygous presence of the minor allele of the marker SNP ID: rs16754 correlates with increased survival rates, especially for the long term survival rates. The median follow-up time for patients who remained alive was 5.3 years (range of 0.2-12 years). 87.5% of patients (n=56) with the minor allele of marker SNP ID: rs16754 (at least one allele) gained a CR compared to 76.2% of patients (n=141) having the major allele (P=0.06). In univariate analysis, patients with the minor allele (at least heterozygously) had a significantly longer RFS (HR=0.48; 95% CI 0.3 to 0.77; 5-years RFS 60% [n=56] vs. 37% [n=141], P=0.002) and significantly longer OS (HR 0.39; 95% CI 0.24 to 0.63; 5-years OS 66% vs. 37%, P<0.001) compared to patients having the major allele homozygously. The positive prognostic value of the minor allele of marker SNP ID: rs16754 occurs in all AML patients. A sub-group analysis using mutation status of NPM1 and FLT3 showed that the positive prognostic value of marker SNP ID: rs16754 is most pronounced in, and preferably restricted to, high-risk mutation status patients (*NPM1*^{wildtype}/*FLT3*-ITD^{uegative} (wild-type FLT3), or *NPM1*^{wildtype}/*FLT3-ITD*^{positive} (FLT3-ITD), or *NPM1*^{mutated}/*FLT3-ITD*^{positive}; RFS, n=120; HR=0.43; 95% CI 0.23 to 0.79, P=0.005; OS, n=159, HR=0.38; 95% CI 0.21 to 0.67, P=0.001), which patients form the preferred group of high-risk patients. In patients with low-risk *NPM1* ^{mutated}/*FLT3*-ITD^{negative} status no significant difference in RFS could be detected for SNP rs16754 alleles, however, carriers of minor allele of SNP rs16754 showed a trend towards improved OS (see also Fig. 1).

The results shown in Figure 1 for patients who are classified as low risk in accordance with mutant-type NPM1 and/or wild-type FLT3 for C) the relapse-free survival and for D) the overall survival, for patients who have the minor allele of marker SNP ID: rs16754 at least heterozygously (AG/AA) in comparison to patients having the major allele of the marker SNP ID: rs16754 homozygously demonstrate that the positive prognosis for carriers of the minor allele of marker SNP ID: rs16754 in chemotherapy also occurs in low risk patients (RFS, n=74, HR=0.6, 95% CI 0.27 to 1.33, P=0.21; OS, n=86, HR=0.42, 95% CI 0.18 to 1.02, P=0.05), albeit to a lesser extent than in high-risk patients.

The positive prognosis of the presence of the minor allele of marker SNP ID: rs16754 on RFS and OS was also found when restricting the analysis to NPM1/FLT3-IDTD high-risk patients with wild-type CEBPA (RFS, n=91, HR=0.43, 95% CI 0.22 to 0.83; P=0.01; OS, n=126, HR=0.39, 95% CI 0.22 to 0.71, P=0.002), which patients also form a preferred group of high-risk AML patients in the invention.

The correlation of the presence of the at least heterozygous minor allele of marker SNP ID: rs16754 with a positive prognosis of chemotherapy, in the present study approx. 13% (33 from 243) of AML patients could not be identified by any other marker or mutation. Presence of the minor allele of SNP rs16754 is an independent predictor of relapse-free survival (RFS) (HR 0.49, 95% CI 0.3 to 0.81, P=0.005) and overall survival (OS) (HR 0.44, 95% CI 0.27 to 0.74, P=0.002), as e.g. determined in multivariate analysis.

Therefore, according to the present knowledge, the prognostic value of the presence of the minor allele of the marker SNP ID: rs16754 for chemotherapy of high-risk leukemia patients is unique. Accordingly, the use of the analysis of the marker SNP ID: rs16754 for the selection of the therapeutic treatment of high-risk leukemia patients, and the use of chemotherapeutical agents for use in the production of a pharmaceutical composition in the treatment of leukemia in high-risk patients who at least heterozygously carry the minor allele of the marker SNP ID: rs16754 could not be expected from the analysis of other genetic markers known to be analysed for identifying a suitable therapy against leukemia.

An intent-to-treat analysis of the patients having the minor allele of marker SNP ID: rs16754 for whom information on a related donor for stem cell transplantation showed that allogeneic stem cell transplantation had no significant impact on OS in these patients.

For the purposes of this invention, reference to WT1 mutations denotes mutations in the WT1 gene, except for the marker SNP ID: rs16754. Accordingly, WT1 mutations include aberrations in the nucleotide sequence which result in a modified gene product. In order to take account of natural variation in WT1 gene sequence, WT1 mutations preferably are gene aberrations that occur in AML patients but are not found in healthy humans. Generally, aberrations occurring in the WT1 gene of healthy humans as well as in AML patients are considered polymorphisms, and in the case of the amino acid sequence encoded being without changes considered silent polymorphisms, which are not necessarily linked to AML.

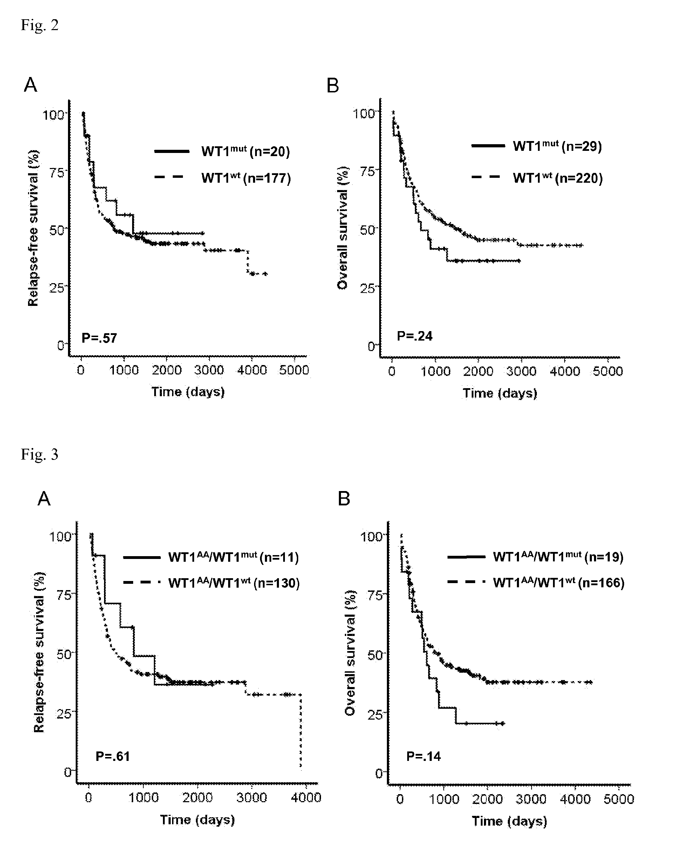

Figure 2 shows that the mutation status of WT1 does not have a significant impact on CR (P=0.15, 69% for mutated WT1 vs. 80.5% for non-mutated WT1), nor on RFS (HR = (0.82, 95% CI 0.42-1.63, 5 years RFS 48% for mutated WT1 vs. 43% for non-mutated WT1), nor on OS (HR = 1.36, 95% CI 0.81-2.26, 5 years OS 36% for mutated WT1 vs. 46% for non-mutated WT1).

The results of Figure 3 show that for patients being homozygous for the major allele of marker SNP ID: rs16754 when analysed for the prognostic value of WT1 mutations, the RFS and OS were essentially the same when comparing mutant WT1 genotypes to wild-type WT1 genotypes. This result indicates that the favourable prognostic value of the minor allele of the marker SNP ID: rs16754 is not masked by other mutations in WT1 (RFS, HR=0.81, 95% CI 0.35 to 1.85; OS, HR=1.5, 95% CI 0.87 to 2.67).

Further, it was found that the expression level of WT1 does not correlate with the prognostic value of the alleles of marker SNP ID: rs16754.

Eighty-one percent of patients with low expression of the WT1 gene achieved a CR compared to 77% of patients with high *WT1* expression (P=0.4). Overall survival but not RFS of patients with high *WT1* expression was significantly shorter compared to patients with low *WT1* expression (RFS, n=186, HR=1.35; 95% CI 0.91 to 1.99; OS, HR=1.47; 95% CI 1.03 to 2.11; 5-years OS 37% ν 51%, respectively). For patients in whom *WT1* expression was measured in peripheral blood (n=66) or in bone marrow blasts (n=163) were analyzed separately, no significant difference in OS was found in either the peripheral blood or bone marrow group (data not shown).

In multivariate analysis for OS, *WT1* SNP rs16754, it was found that the minor allele predicted favourable prognosis (HR 0.44, 95% CI 0.27 to 0.74, P=0.002) independently from *NPM1*/*FLT3* mutation status, *CEBPA* mutation status, *WT1* expression status, age, WBC count, and platelet count. In multivariate analysis for RFS, presence of one at least one minor allele of SNP rs16754 was a favourable prognostic factor (HR 0.49, 95% CI 0.3 to 0.81, P=0.005), which is independent form *NPM1*/*FLT3* mutation status, *CEBPA* mutation status, WBC count, and platelet count. By contrast *WT1* expression above or below the median was not an independent prognostic marker for OS.

The positive prognostic value of the minor allele of the marker SNP rs16754 for RFS and OS for treatment by chemotherapy only, and hence the use of chemotherapeutic agents for the production of pharmaceutical compositions for treatment of the group of AML patients carrying this minor allele, was confirmed in approx. 13% (33 patients) of the entire patient group, and especially stronger in AML high risk NPM1/FLT3-ITD patients than in low risk NPM1/FLT3-ITD patients. No other favourable genetic marker was identified which has the correlation of positive prognostic value for chemotherapy in AML patients as found for the minor allele of the marker SNP rs16754.

Analysis of healthy volunteers in respect of alleles of the marker SNP rs16754 in comparison to alleles found in AML patients did not reveal any correlation between allele distribution and AML. Currently, no phenotypic effect of the minor or major allele of the marker SNP rs16754, respectively, could be found, e.g. both alleles result in the same amino acid sequence of the translation product. As a consequence of these two observations, it is currently assumed that presence of the major allele of the marker has an impact on the response to chemotherapeutical agents, but no direct effect on the development of leukemia.

Accordingly, in addition to the analytical method for the determination of the allele of the marker SNP ID: rs16754 in a leukemia patient, the present invention also relates to the use of chemotherapeutical agents, especially of idarubicin, etoposide , cytarabine, high-dose AraC, fludarabine, G -CSF, and/or daunorubicin for use in the production of a pharmaceutical composition for use in leukemia therapy in high risk CN-AML patients, who are at least heterozygous for the minor allele of the marker SNP ID: rs16754.

As it has been shown in the invention that the absence of the transcript of the WT1 gene of the major allele of the marker SNP ID: rs16754 is linked to the successful treatment using chemotherapeutical agents, suppression of the major allele WT1 mRNA further increases the prospects of the use of chemotherapeutical agents in the treatment of AML patients. Accordingly, the invention further provides for a pharmaceutical composition containing a nucleotide sequence encoding or containing an RNA having a nucleic acid sequence which is reverse complementary to at least a section of the WT1 gene containing the major allele of the marker SNP ID: rs16754, and to the use of a nucleotide sequence encoding or containing an RNA having a nucleic acid sequence reverse complementary to at least a section of the WT1 gene containing the major allele of the marker SNP ID: rs16754 for the production of a pharmaceutical composition, respectively, especially for use in the treatment of AML. Preferably, the nucleic acid sequence encoding or containing an RNA having a nucleic acid sequence which is reverse complementary to at least a section of the WT1 gene containing the major allele of the marker SNP ID: rs16754 is combined with a chemotherapeutical agent, e.g. for use in the treatment of AML patients who carry the major allele of the marker homozygously.

On the other hand, the invention also allows to identify high risk CN-AML patients, who are homozygous for the major allele of the marker as not suitable for chemotherapy, as the presence of the major allele in a homozygous state indicates a lack of effectiveness of standard chemotherapy. Accordingly, the invention provides for the analysis of patients in respect of the marker, for assigning these patients to intensive treatment, including stem cell therapy.

## Claims

1. Use of a process for the analysis of the allele of the marker Ref SNP ID rs16754 in a nucleic acid sample obtained from a human suffering from leukemia in the selection of a therapy.

2. Use according to claim 1, **characterized in that** the leukemia is selected from AML or ALL.

3. Use according to one of the preceding claims, **characterized in that** the human has been identified to have a high-risk AML genotype.

4. Use according to claim, **characterized in that** the high-risk AML genotype is selected from the group of genotypes of wild-type NPM1 in combination with wild-type FLT3, wild-type NPM1 in combination with an FLT3 - ITD mutation, and a NPM1 mutation in combination with an FLT3 - ITD mutation.

5. Use according to one of the preceding claims, **characterized in that** the human is identified to have cytogenetically normal AML (CN-AML).

6. Use according to one of the preceding claims, **characterized in that** the patient carries an at least heterozygous allele with G in the marker Ref SNP rs16754, and the therapy consists of chemotherapy.

7. Use according to one of claims 1 to 5, **characterized in that** the patient homozygously carries the allele with A in the marker Ref SNP rs16754, and the therapy comprises intensive chemotherapy including stem cell therapy.

8. Use of the analysis of the genotype of a patient in the selection of a therapy for a leukemia patient, **characterized in that** the analysis comprises the determination of the allele of the marker ref SNP rs16754 in a nucleic acid sample obtained from the patient.

9. Use of a kit of parts containing oligonucleotides which are complementary to at least a section of the human WT1 gene corresponding to SEQ ID No. 1 for the analysis of the allele of the marker Ref SNP ID rs16754 in a nucleic acid sample obtained from a human suffering from leukemia, for selecting a therapy.

10. Use according to claim 9, **characterized in that** the allele of the marker Ref SNP ID rs16754 is G and the therapy consists of chemotherapy.

11. Use of a chemotherapeutical agent for the production of a pharmaceutical composition for the treatment of a human leukemia patient, **characterized in that** the patient carries an at least heterozygous allele with G in the marker Ref SNP rs16754.

12. Use according to claim 11, **characterized in that** the leukemia patient is identified to have a high-risk AML genotype.

13. Use according to claim 11 or 12, **characterized in that** the high-risk AML genotype is selected from the group of genotypes of wild-type NPM1 in combination with wild-type FLT3, wild-type NPM1 in combination with an FLT3 - ITD mutation, and a NPM1 mutation in combination with an FLT3 - ITD mutation, and **in that** the high risk leukemia patient is identified to have cytogenetically normal AML (CN-AML).

14. Pharmaceutical compound containing at least one chemotherapeutical active compound for use in the therapy of leukemia, **characterized in that** the pharmaceutical compound is accompanied by instructions for use in the treatment of AML patients only who carry the allele G of marker Ref SNP ID rs16754.

15. Pharmaceutical compound according to claim 14, **characterized in that** the instructions define the AML patients as high-risk AML genotype.
